(19) Europäisches Patentamt
European Patent Office
Office européen des brevets

(11) Veröffentlichungsnummer: **0 306 868 B1**

# (12) EUROPÄISCHE PATENTSCHRIFT

(45) Veröffentlichungstag der Patentschrift: **27.12.91**

(51) Int. Cl.$^5$: **C07D 233/90**

(21) Anmeldenummer: **88114425.7**

(22) Anmeldetag: **03.09.88**

## (54) Verfahren zur Herstellung von 1-Alkylimidazolen.

(30) Priorität: **05.09.87 DE 3729852**

(43) Veröffentlichungstag der Anmeldung:
**15.03.89 Patentblatt 89/11**

(45) Bekanntmachung des Hinweises auf die Patenterteilung:
**27.12.91 Patentblatt 91/52**

(84) Benannte Vertragsstaaten:
**CH DE FR GB IT LI NL**

(56) Entgegenhaltungen:
**EP-A- 0 172 407**

(73) Patentinhaber: **BASF Aktiengesellschaft**
**Carl-Bosch-Strasse 38**
**W-6700 Ludwigshafen(DE)**

(72) Erfinder: **Koehler, Hermann, Dr.**
**Roentgenstrasse 7**
**W-6711 Beindersheim(DE)**
Erfinder: **Dockner, Toni, Dr.**
**Grossgasse 6**
**W-6701 Meckenheim(DE)**
Erfinder: **Karn, Helmut**
**Sternstrasse 120**
**W-6700 Ludwigshafen(DE)**

**EP 0 306 868 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Vorliegende Erfindung betrifft ein neues und verbessertes Verfahren zur Herstellung von 1-Alkylimidazolen durch Umsetzung von Imidazolen mit Dialkylsulfaten bei erhöhten Temperaturen, indem man die Umsetzung in Gegenwart einer Carbonsäure und/oder eines Carbonsäureanhydrids vornimmt.

Aus JACS 71, 644-647 (1949) ist bekannt, daß man aus N-Methylglycinmethylester-hydrochlorid in einer 3-stufigen Reaktionssequenz 1-Methylimidazol-5-carbonsäuremethylester in einer Gesamtausbeute von 35 % erhält.

Weiterhin ist aus Bull. Chem. Soc. Japan 53, 557-558 (1980) die Herstellung von 1-Methylimidazol-5-carbonsäure durch selektive Decarboxylierung von 1-Methylimidazol-4,5-dicarbonsäure bekannt.

Aus J. Chem. Soc. 21-23 (1928) ist die Umsetzung von Imidazol-4(5)-carbonsäuremethylester mit Dimethylsulfat in Abwesenheit eines Lösungsmittels bekannt, die zu 1-Methylimidazol-5-carbonsäuremethylester in 37-%iger Ausbeute führt.

Der Erfindung lag daher die Aufgabe zugrunde, einen neuen bzw. besseren Zugang zu 1-Alkylimidazolen zu finden.

Demgemäß wurde ein neues und verbessertes Verfahren zur Herstellung von 1-Alkylimidazolen der allgemeinen Formel I

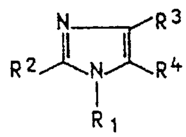

(I),

in der

| | |
|---|---|
| $R^1$ | $C_1$- bis $C_{20}$-Alkyl, |
| $R^2$, $R^3$ | Wasserstoff, $C_1$- bis $C_{20}$-Alkyl, Phenyl, 1-Naphthyl, 2-Naphthyl, 1-Anthryl, 2-Anthryl, 9-Anthryl, $C_7$- bis $C_{10}$-Phenalkyl oder $C_7$- bis $C_{10}$-Alkylphenyl, |
| $R^4$ | -COOH, $-COOR^5$, $-CONH_2$ oder Cyano und |
| $R^5$ | $C_1$- bis $C_8$-Alkyl |

bedeuten, durch Umsetzung von Imidazolen der allgemeinen Formel II

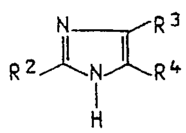

(II),

mit Dialkylsulfaten der allgemeinen Formel III

$(R^1O)_2SO_2$ (III)

bei erhöhten Temperaturen gefunden, welches dadurch gekennzeichnet ist, daß man die Umsetzung von III mit II im Molverhältnis von 0,4:1 bis 10:1 in Gegenwart einer Carbonsäure und/oder eines Carbonsäureanhydrids vornimmt.

Die 1-Alkylimidazole [ sind nach folgender Methode erhältlich:
Die Umsetzung erfolgt zwischen einem Imidazol II und einem Dialkylsulfat bei erhöhten Temperaturen in Gegenwart einer Carbonsäure und/oder eines Carbonsäureanhydrids nach folgender Reaktionsgleichung:

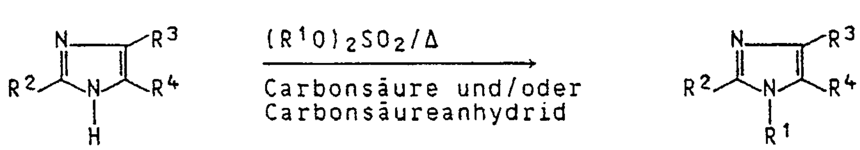


(II) (I)

Die Reaktion erfolgt vorzugsweise bei 40 bis 220 °C, besonders bevorzugt bei 80 bis 150 °C und

Normaldruck (1 bar), kann aber auch bei erhöhtem Druck bis 10 bar und auch bei erniedrigtem Druck bis 0,1 bar durchgeführt werden.

Das Verfahren kann kontinuierlich oder diskontinuierlich durchgeführt werden.

Die Imidazol-4(5)-carbonsäuren sind überwiegend bekannt und können nach dem in der DE-A-34 27 136 beschriebenen Verfahren hergestellt werden. Die entsprechenden Imidazol-4 (5)-carbonsäureester und -carboxamide lassen sich nach allgemein bekannten Methoden aus den Imidazol-4 (5) -carbonsäuren herstellen. Die Imidazol-4 (5)-cyanoverbindungen sind aus den Imidazol4 (5)-carboxamiden durch Umsetzung mit wasserbindenden Mitteln, z.B. Phosphorpentachlorid oder Phosphoroxitrichlorid erhältlich.

Das Molverhältnis von Dialkylsulfat: Imidazol II beträgt von 0,4:1 bis 10:1, vorzugsweise von 0,5:1 bis 3:1.

Als Carbonsäuren eignen sich unverzweigte und verzweigte aliphatische $C_1$-$C_8$-Carbonsäuren, vorzugsweise unverzweigte und verzweigte $C_1$-$C_6$-Carbonsäuren, wie Ameisensäure, Essigsäure, Propionsäure, n-Buttersäure, iso-Buttersäure, Valeriansäure, iso-Valeriansäure, Pivalinsäure und Capronsäure, besonders bevorzugt Ameisensäure, Essigsäure und Propionsäure. Als Carbonsäureanhydride eignen sich unverzweigte und verzweigte aliphatische $C_4$-$C_{16}$-Carbonsäureanhydride, vorzugsweise unverzweigte und verzweigte aliphatische $C_4$-$C_{12}$-Carbonsäureanhydride, wie Acetanhydrid, Propionsäureanhydrid, n-Buttersäureanhydrid, iso-Buttersäureanhydrid, Valeriansäureanhydrid, iso-Valeriansäureanhydrid, Pivalinsäureanhydrid und Capronsäureanhydrid, sowie gemischte Anhydride, besonders bevorzugt Acetanhydrid, sowie Gemische aus Carbonsäuren, Anhydriden und/oder gemischten Anhydriden. Die Carbonsäuren sind bevorzugt und die Carbonsäureanhydride besonders bevorzugt.

Die Carbonsäuren und/oder Carbonsäureanhydride werden in solchen Mengen zugesetzt, daß rührbare Reaktionsmischungen entstehen, vorzugsweise 40 bis 2000 ml, besonders bevorzugt 100 bis 500 ml pro Mol Imidazol II. Größere Mengen der Carbonsäure und/oder Carbonsäureanhydride sind i.a. nicht nötig, können aber auch verwendet werden.

Die Reaktionsgemische werden in üblicher Weise aufgearbeitet, z.B. durch Versetzen mit Wasser, Trennen der Phasen und Säulenchromatographie. Die Verbindungen der Formel I fallen teilweise in Form farbloser oder schwach bräunlich gefärbter, zäher Öle an, die sich durch längeres Erwärmen unter vermindertem Druck auf mäßig erhöhte Temperatur ("Andestillieren") von den letzten flüchtigen Anteilen befreien und auf diese Weise reinigen lassen. Erhält man die Verbindungen der Formel I in kristalliner Form, so kann ihre Reinigung durch Umkristallieren erfolgen.

Die Substituenten $R^1$ bis $R^5$ in den Formeln I, II und III haben folgende Bedeutungen:

$R^1$ unverzweigtes und verzweigtes $C_1$-$C_{20}$-Alkyl, bevorzugt unverzweigtes oder verzweigtes $C_1$-$C_{12}$-Alkyl, wie Methyl, Ethyl, n-Propyl, isoPropyl, n-Butyl, iso-Butyl, sec. -Butyl, tert.-Butyl, n-Pentyl, iso-Pentyl, sec. -Pentyl, tert.-Pentyl, neo-Pentyl, 1 ,2-Dimethyl-propyl, n-Hexyl, iso-Hexyl, sec. -Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl,

$R^2$ ,$R^3$ unabhängig voneinander

- Wasserstoff,
- unverzweigtes und verzweigtes $C_1$-$C_{20}$-Alkyl, bevorzugt unverzweigtes oder verzweigtes $C_1$-$C_{12}$-Alkyl, wie Methyl, Ethyl, n-Propyl, n-Butyl, iso-Butyl, sec. -Butyl, tert.-Butyl, n-Pentyl, iso-Pentyl, sec. -Pentyl, tert.-Pentyl, neo-Pentyl, 1 ,2-Dimethylpropyl, n-Hexyl, iso-Hexyl, sec. -Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl,
- Aryl, bevorzugt Phenyl, 1-Naphthyl, 2-Naphthyl, 1-Anthryl, 2-Anthryl und 9-Anthryl, besonders bevorzugt Phenyl, unverzweigtes und verzweigtes $C_7$-$C_{10}$-Phenylalkyl, wie Benzyl, 1-Phenethyl, 2-Phenethyl, 1-Phenyl-n-propyl, 2-Phenyl-n-propyl, 3-Phenyl-n-propyl,
- unverzweigtes oder verzweigtes $C_7$-$C_{10}$-Alkylphenyl, wie 2-Methylphenyl, 3-Methylphenyl, 4-Methylphenyl, 2-Ethylphenyl, 3-Ethylphenyl, 4-Ethylphenyl, 2-n-Propylphenyl, 3-n-Propylphenyl, 4-n-Propylphenyl, 2-iso-Propylphenyl, 3-iso-Propylphenyl, 4-iso-Propylphenyl, 2-n-Butylphenyl, 3-n-Butylphenyl, 4-n-Butylphenyl, 2-iso-Butylphenyl, 3-iso-Butylphenyl, 4-iso-Butylphenyl, 2-sec. -Butylphenyl, 3-sec. -Butylphenyl, 4-sec. -Butylphenyl, 2-tert.-Butylphenyl, 3-tert. -Butylphenyl, 4-tert. -Butylphenyl, 2,4-Dimethylphenyl, 3,4-Dimethylphenyl, 2,6-Dimethylphenyl, 3,4-Diethylphenyl, 3,4,5-Trimethylphenyl, 2,4,6-Trimethylphenyl und 2-Ethyl-4-methylphenyl,

$R^4$

- -COOH,
- -COOR$^5$,

- $-CONH_2$,
- Cyano und

$R^5$

- unverzweigtes und verzweigtes $C_1$-$C_8$-Alkyl, vorzugsweise unverzweigtes und verzweigtes $C_1$-$C_4$-Alkyl wie Methyl, Ethyl, n-Propyl, isoPropyl, n-Butyl, iso-Butyl, sec. -Butyl und tert.-Butyl.

Für den Fall, daß $R^4$ in den Verbindungen I und II für -COOH steht, empfiehlt es sich die Verbindungen I nicht auf der Stufe der freien Carbonsäure, sondern auf der Stufe ihrer Ester ($-COOR^5$) zu isolieren, es sei denn die Verbindungen I mit der freien Carboxylgruppe werden benötigt.

Die Verbindungen der Formel I sind Zwischenprodukte für die Synthese von Wirkstoffen.

Durch die folgenden Beispiele wird die Erfindung näher erläutert.

Beispiele

Beispiel 1

Herstellung von 1-Methylimidazol-5-carbonsäure

Zu einer Lösung von 112 g (1 mol) Imidazol-4(5)-carbonsäure in 200 ml Acetanhydrid wurden bei 110 °C innerhalb von 1 Stunde 252 g (2 mol) Dimethylsulfat zugetropft, 3 Stunden unter Rückfluß erhitzt und anschließend die flüchtigen Anteile abdestilliert.

Zur Charakterisierung wurde die 1-Methylimidazol-5-carbonsäure wie folgt in ihren Methylester überführt:

Der zuvor erhaltene Rückstand wurde mit 300 ml Methanol und 150 ml konz. Schwefelsäure 2 Stunden unter Rückfluß erhitzt, anschließend das Methanol abdestilliert, mit 250 ml Wasser verdünnt und mit konz. Ammoniak neutralisiert. Nach Extraktion mit Methylenchlorid, Trocknung und Entfernen des Lösungsmittels i. Vak. erhielt man eine Gesamtausbeute von 105,1 g (75 %) 1-Methylimidazol-5-carbonsäuremethylester; Fp. 44-46 °C.

$^1$H-NMR ($CDCl_3$): 3,86 ppm (s, 3 H), 3,91 ppm (s, 3 H), 7,58 ppm (s, 1 H), 7,72 ppm (s, 1 H).

$^{13}$C-NMR ($CDCl_3$): 34,0 ppm, 51,4 ppm, 123,1 ppm, 137,6 ppm, 142,6 ppm, 160,8 ppm (alle Signale s).

Beispiel 2

Herstellung von 1-Ethylimidazol-5-carbonsäure

Zu einer Lösung von 112 g (1 mol) Imidazol-4(5)-carbonsäure in 200 ml Acetanhydrid wurden bei 110 °C innerhalb von 1 Stunde 308 g (2 mol) Diethylsulfat zugetropft, 3 Stunden unter Rückfluß erhitzt und anschließend die flüchtigen Anteile abdestilliert.

Zur Charakterisierung wurde die 1-Ethylimidazol-5-carbonsäure wie folgt über ihren Methylester in das Hydrochlorid der Carbonsäure überführt:

Der zuvor erhaltene Rückstand wurde mit 300 ml Methanol und 150 ml konz. Schwefelsäure 2 Stunden unter Rückfluß erhitzt, anschließend das Methanol abdestilliert, mit 250 ml Wasser verdünnt und mit konz. Ammoniak neutralisiert. Nach Extraktion mit Methylenchlorid, Trocknung und Entfernen des Lösungsmittels i. Vak. erhielt man den 1-Ethylimidazol-5-carbonsäuremethylester, der durch 4-stündiges Erhitzen mit 6 N Salzsäure, anschließendem Eindampfen und Umkristallisieren aus Methanol in einer Gesamtausbeute von 95,3 g (54 %) in das 1-Ethylimidazol-5-carbonsäure-hydrochlorid; Fp.: 238-239 (Zers.) überführt wurde.

$C_6H_9N_2O_2$ Cl: Ber: C 40,8; H 5,1; N 15,9; Cl 20,1.

(176,6) Gef: C 40,8; H 5,1; N 15,7; Cl 20,2.

$^1$H-NMR (DMSO-$d_6$): 1,45 ppm (t, 3 H), 4,53 ppm (q, 2 H), 8,35 ppm (s, 1 H), 9,55 ppm (s, 1 H).

$^{13}$C-NMR (DMSO-$d_6$): 15,5 ppm, 43,7 ppm, 124,0 ppm, 126,5 ppm, 138,6 ppm, 158,9 ppm (alle Signale s).

Beispiel 3

Herstellung von 1,2-Dimethylimidazol-5-carbonsäure

126 g (1 mol) 2-Methylimidazol-4(5)-carbonsäure werden analog Beispiel 1 umgesetzt und ebenfalls zur Charakterisierung in den entsprechenden Methylester überführt. Man erhielt 103,3 g (67 %) 1,2-Dimethylimidazol-5-carbonsäuremethylester; Fp.: 38 - 40 °C.

$C_7H_{10}N_2O_2$: Ber: C 54,5; H 6,5; N 18,2.
(154,2) Gef: C 54,2; H 6,6; N 18,0.
$^1$H-NMR ($CDCl_3$): 2,42 ppm (s, 3 H), 3,83 ppm (s, 6 H), 7,62 ppm (s, 1 H).
$^{13}$C-NMR ($CDCl_3$): 13,4 ppm, 32,1 ppm, 51,2 ppm, 122,9 ppm, 136,2 ppm, 150,4 ppm, 160,9 ppm (alle Signale s).

Beispiel 4

Herstellung von 1-Methylimidazol-5-carbonsäuremethylester

Zu einer Lösung von 126 g (1 mol) Imidazol-4(5)-carbonsäuremethylester in 250 ml Acetanhydrid wurden bei 110 °C innerhalb von 1 Stunde 252 g (2 mol) Dimethylsulfat zugetropft und 3 Stunden unter Rückfluß erhitzt. Anschließend wurden die flüchtigen Anteile abdestilliert, der Rückstand mit 250 ml Wasser verdünnt, die Lösung mit konz. wäßrigem Ammoniak neutralisiert und mit Methylenchlorid extrahiert. Nach Trocknung und Entfernung des Lösungsmittels i. Vak. erhielt man 115,0 g (82 %) 1-Methylimidazol-5-carbonsäuremethylester; Fp. 44-46 °C.

**Patentansprüche**

1. Verfahren zur Herstellung von 1-Alkylimidazolen der allgemeinen Formel I

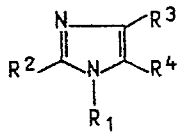

(I),

in der
R[1] $C_1$- bis $C_{20}$-Alkyl,
$R^2$, $R^3$ Wasserstoff, $C_1$- bis $C_{20}$-Alkyl, Phenyl, 1-Naphthyl, 2-Naphthyl, 1-Anthryl, 2-Anthryl, 9-Anthryl, $C_7$- bis $C_{10}$-Phenalkyl oder $C_7$-bis $C_{10}$-Alkylphenyl,
$R^4$ -COOH, -$COOR^5$, -$CONH_2$ oder Cyano und
$R^5$ $C_1$- bis $C_8$-Alkyl
bedeuten, durch Umsetzung von Imidazolen der allgemeinen Formel II

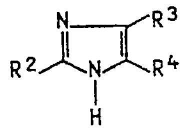

(II)

mit Dialkylsulfaten der allgemeinen Formel III

$(R^1O)_2SO_2$ (III)

bei erhöhten Temperaturen, dadurch gekennzeichnet, daß man die Umsetzung von III mit II im Molverhältnis von 0,4:1 bis 10:1 in Gegenwart einer Carbonsäure und/oder eines Carbonsäureanhydrids vornimmt.

2. Verfahren nach Anspruch 1, dadurch gekennzeichnet, daß man die Umsetzung in einer aliphatischen $C_1$-$C_8$-Carbonsäure und/oder in einem aliphatischen $C_4$-$C_{16}$-Carbonsäureanhydrid vornimmt.

3. Verfahren nach Anspruch 1, dadurch gekennzeichnet, daß man die Umsetzung in einem aliphatischen $C_4$-$C_{12}$-Carbonsäureanhydrid vornimmt.

4. Verfahren nach Anspruch 1, dadurch gekennzeichnet, daß man die Umsetzung in Gegenwart von 40 bis 2000 ml einer Carbonsäure und/oder eines Carbonsäureanhydrids bezogen auf 1 mol eines Imidazols II vornimmt.

## Claims

1. A process for preparing 1-alkylimidazoles of the formula I

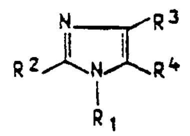

(I)

where

| | |
|---|---|
| $R^1$ | is $C_1$-$C_{20}$-alkyl, |
| $R^2$ and $R^3$ | are each hydrogen, $C_1$-$C_{20}$-alkyl, phenyl, 1-naphthyl, 2-naphthyl, 1-anthryl, 2-anthryl, 9-anthryl, $C_7$-$C_{10}$-phenalkyl or $C_7$-$C_{10}$-alkylphenyl, |
| $R^4$ | is -COOH, -COOR$^5$, -CONH$_2$ or cyano and |
| $R^5$ | is $C_1$-$C_8$-alkyl, |

by reacting imidazoles of the formula II

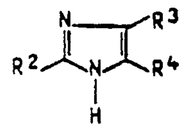

(II)

with dialkyl sulfates of the formula III

$(R^1O)_2SO_2$ (III)

at elevated temperatures, which comprises carrying out the reaction of III with II in the molar ratio of from 0.4:1 to 10:1 in the presence of a carboxylic acid and/or of a carboxylic anhydride.

2. A process as claimed in claim 1, wherein the reaction is carried out in an aliphatic $C_1$-$C_8$-carboxylic acid and/or in an aliphatic $C_4$-$C_{16}$-carboxylic anhydride.

3. A process as claimed in claim 1, wherein the reaction is carried out in an aliphatic $C_4$-$C_{12}$-carboxylic anhydride.

4. A process as claimed in claim 1, wherein the reaction is carried out in the presence of from 40 to 2,000 ml of a carboxylic acid and/or of a carboxylic anhydride based on 1 mol of an imidazole II.

## Revendications

1. Procédé de préparation de 1-alkylimidazoles de formule générale I

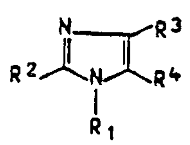

(I),

dans laquelle

$R^1$ est un radical alkyle en $C_1$ à $C_{20}$,

$R^2$, $R^3$ représentent chacun un hydrogène, un radical alkyle en $C_1$-$C_{20}$, le radical phényle, 1-naphtyle, 2-naphtyle, 1-anthryle, 2-anthryle, 9-anthryle, un radical phénalkyle en $C_7$ à $C_{10}$ ou (alkyle en $C_7$ à $C_{10}$)phényle,

$R^4$ est -COOH, -COOR$^5$, -CONH$_2$ ou cyano, et

$R^5$ est un radical alkyle en $C_1$ à $C_8$,

par réaction d'imidazoles de formule générale II

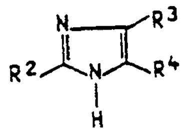

(II)

avec des sulfates de dialkyle de formule générale III

$(R^1O)_2SO_2$ (III)

à température élevée, caractérisé en ce qu'on met en oeuvre la réaction du composé III avec le composé II selon un rapport en moles de 0,4:1 à 10:1 en présence d'un acide carboxylique et/ou d'un anhydride d'un acide carboxylique.

**2.** Procédé selon la revendication 1, caractérisé en ce qu'on met en oeuvre la réaction dans un acide carboxylique aliphatique en $C_1$-$C_8$ et/ou dans un anhydride d'un acide aliphatique carboxylique en $C_4$-$C_{16}$.

**3.** Procédé selon la revendication 1, caractérisé en ce qu'on met en oeuvre la réaction dans un anhydride d'un acide carboxylique aliphatique en $C_4$-$C_{12}$.

**4.** Procédé selon la revendication 1, caractérisé en ce qu'on met en oeuvre la réaction en présence de 40 à 2000 ml d'un acide carboxylique et/ou d'un anhydride d'un acide carboxylique, par mole d'un imidazole II.